# EUROPÄISCHE PATENTSCHRIFT

(11) **EP 2 914 313 B1**
(45) Veröffentlichungstag und Bekanntmachung des Hinweises auf die Patenterteilung: **03.01.2018**
(21) Anmeldenummer: 13789745.0
(22) Anmeldetag: 31.10.2013
(51) Int. Cl.: A61M 1/06

(54) **ABPUMPEINHEIT ZUM ABPUMPEN VON MUTTERMILCH**
PUMPING UNIT FOR PUMPING BREAST MILK
UNITÉ DE TIRAGE DU LAIT PERMETTANT LE TIRAGE DU LAIT MATERNEL

(30) Priorität: 05.11.2012 US 201261722563 P; 14.03.2013 US 201313828333
(43) Veröffentlichungstag der Anmeldung: 09.09.2015
(73) Patentinhaber: Medela Holding AG, 6340 Baar (CH)
(72) Erfinder: SCHLIENGER, André, CH-8933 Maschwanden (CH); RIGERT, Mario, CH-6033 Buchrain (CH); FURRER, Etienne, CH-6332 Hagendorn (CH); GILBERT, Deanna, Arlington Heights, Illinois 60005 (US)
(74) Vertreter: Clerc, Natalia
(86) Internationale Anmeldenummer: PCT/EP2013/072827
(87) Internationale Veröffentlichungsnummer: WO 2014/068066

(56) Entgegenhaltungen:
- EP-A1- 1 034 807
- US-A- 4 680 028
- US-A- 5 571 084
- US-A- 6 073 788
- US-A- 6 092 680
- US-A1- 2009 171 270

## Beschreibung

### TECHNISCHES GEBIET

Die vorliegende Erfindung betrifft eine Abpumpeinheit zur Verwendung in einer Vorrichtung zum Abpumpen von menschlicher Muttermilch gemäss Oberbegriff des Patentanspruchs 1, eine Brusthaube gemäss Oberbegriff des Patentanspruchs 6, einen Milchsammelbehälter gemäss Oberbegriff des Patentanspruchs 7 sowie eine Kopplungseinheit gemäss Oberbegriff des Patentanspruchs 8.

### STAND DER TECHNIK

Vorrichtungen zum Abpumpen von menschlicher Muttermilch sind hinlänglich bekannt. Grundsätzlich gibt es zwei verschiedene Typen: die ersten werden manuell bedient, d.h. der für das Abpumpen notwendige Unterdruck wird durch manuelle Betätigung der Vakuumpumpe erzeugt. Bei den zweiten Typen ist die Vakuumpumpe mit einem Elektromotor betrieben.
In diesen Vorrichtungen werden Abpumpeinheiten eingesetzt, welche eine Brusthaube und einen damit verbundenen Milchsammelbehälter umfassen. Die Brusthaube weist üblicherweise einen Trichter zur dichtenden Anlage an die Mutterbrust auf. Der Behälter ist mit der Brusthaube lösbar verbunden, entweder direkt oder über eine Schlauchverbindung. Ist der Behälter formstabil, insbesondere steif ausgebildet, so kann er sowohl als Vorratsbehälter für die Milch wie auch als Unterteil einer Milchflasche zur Verabreichung der Milch eingesetzt werden. Derartige formstabile Behälter sind einfach in ihrer Handhabung, insbesondere wenn sie selbststehend ausgebildet sind. In anderen Ausführungsformen sind derartige Behälter als weiche Beutel ausgebildet. Nachteilig bei der Verwendung von weichen Beuteln ist, dass sie vor dem dichten Verschliessen sehr sorgfältig behandelt werden müssen, damit keine Milch ausfliesst. Zudem muss die Milch vor Verabreichung an ein Baby in einen anderen Behälter umgegossen werden.

US 2004/0087898 und WO 2008/057218 offenbaren Abpumpeinheiten mit einem formstabilen Milchsammelbehälter, welcher über eine Schraubverbindung mit der Brusthaube verbunden ist. Der Winkel zwischen der Längsmittelachse der Brusthaube und der Längsmittelachse des Milchsammelbehälters ist grösser als 90°.

US 2008/0262420 offenbart eine Brusthaube mit einer Milchsammelkammer. Diese Brusthaube lässt sich in einem Büstenhalter befestigen. Von der Brusthaube führt eine flexible Leitung zu einem Milchsammelbehälter, welcher in einem Gürtel angeordnet ist.

US 7 223 255 und WO 2008/137678 zeigen Brusthauben mit integrierter Vakuumpumpe sowie einen Milchsammelbeutel, welcher über eine Leitung mit der Brusthaube verbunden ist.

EP 1 034 807 A1 und US 2009/0171270 offenbaren weitere Abpumpeinheiten aus dem Stand der Technik.

### DARSTELLUNG DER ERFINDUNG

Es ist eine Aufgabe der Erfindung, eine Abpumpeinheit mit einem formstabilen oder annähernd formstabilen Milchsammelbehälter zu schaffen, welche möglichst kompakt und platzsparend ausgebildet ist und welche der Mutter einen optimalen Komfort beim Abpumpen ermöglicht.

Diese Aufgabe lösen eine Abpumpeinheit mit den Merkmalen des Anspruchs 1, eine Brusthaube mit den Merkmalen des Anspruchs 6, ein Milchsammelbehälter mit den Merkmalen des Anspruchs 7 sowie eine Kopplungseinheit mit den Merkmalen des Anspruchs 8.

Die erfindungsgemässe Abpumpeinheit zur Verwendung in einer Vorrichtung zum Abpumpen von menschlicher Muttermilch weist eine Brusthaube zur Anlage an eine Mutterbrust und einen formstabilen oder annähernd formstabilen Milchsammelbehälter zur Aufnahme der abgepumpten Muttermilch auf. Die Brusthaube weist eine erste Achse, insbesondere eine Längsmittelachse, und der Milchsammelbehälter weist eine zweite Achse, insbesondere eine Längsmittelachse auf. Der Milchsammelbehälter ist lösbar an der Brusthaube befestigbar. Vorzugsweise wird die Brusthaube vom Milchsammelbehälter getragen oder umgekehrt. Die erste Achse ist im befestigten Zustand des Milchsammelbehälters in einem Winkel zur zweiten Achse angeordnet. Erfindungsgemäss ist der Milchsammelbehälter im befestigten Zustand relativ zur Brusthaube bewegbar, wodurch der Winkel zwischen der ersten und der zweiten Achse veränderbar ist.

Da sich der Winkel zwischen Milchsammelbehälter und Brusthaube verändern lässt, sich vorzugsweise beliebig einstellen lässt, kann er in Hinblick auf die individuellen anatomischen Gegebenheiten der Mutterbrust angepasst werden. Die Mutter kann vor dem Abpumpen, vorzugsweise auch während des Abpumpens, den für sie optimalen Winkel einstellen und bei Lageveränderung auch jederzeit und beliebig wieder ändern.

Grosse Brüste benötigen einen anderen Winkel als kleinere Brüste; dasselbe gilt für schlanke und beleibte Frauen. Dank dieser Anpassungsfähigkeit lässt sich der Milchsammelbehälter relativ nahe an den Körper und an der Brusthaube anordnen. Ein langer Zwischenbereich zwischen Brusthaube und Behälter ist nicht notwendig. Die Abpumpeinheit lässt sich somit kompakt und platzsparend ausbilden.

Dank der Veränderbarkeit der relativen Lage des Behälters zur Brusthaube lässt sich die Brusthaube sogar unter einem Büstenhalter tragen, obwohl ein formstabiler oder gar steifer Sammelbehälter in unmittelbarer Nähe zu ihr angeordnet ist. Ein sogenannter "Hands-Free" Betrieb, bei welchem weder Sammelbehälter noch Brusthaube von Hand gehalten werden müssen, ist somit möglich.

Vorzugsweise ist der Sammelbehälter über eine einzige Schnittstelle mit der Brusthaube verbunden. Diese Schnittstelle dient vorzugsweise zugleich als Verbindungskanal, um die Milch von der Brusthaube in den Sammelbehälter zu leiten.

In einer bevorzugten Ausfuhrungsform sind ein erstes und ein zweites Kopplungsteil vorhanden, welche gemeinsam die Relativbewegung ermöglichen, wobei das erste Kopplungsteil an der Brusthaube und das zweite Kopplungsteil am Milchsammelbehälter angeordnet ist. Vorzugsweise weist dabei der Milchsammelbehälter ein Behälterteil und einen diesen Behälterteil verschliessenden Deckel auf, wobei das zweite Kopplungsteil am Deckel angeordnet ist. Die Kopplungsteile bilden ein Gelenk und dienen als Verbindungsmittel, welche den Milchsammelbehälter an der Brusthaube befestigen.

Diese Kopplungsteile dienen in einer bevorzugten Ausfuhrungsform auch als Milchleitung. Hierfür ist ein Verbindungskanal zwischen Brusthaube und Milchsammelbehälter vorhanden, um Milch von der Brusthaube in den Milchsammelbehälter zu leiten, wobei dieser Verbindungskanal durch die zwei Kopplungsteile verläuft. In einer anderen Ausführungsform verläuft dieser Verbindungskanal jedoch ausserhalb der zwei Kopplungsteile.

In einer bevorzugten Ausführungsform sind die zwei Kopplungsteile Bestandteile des Milchsammelbehälters bzw. der Brusthaube. Vorzugsweise ist das erste Kopplungsteil einstückig an einem Teil der Brusthaube angeformt und/oder das zweite Kopplungsteil ist einstückig an einem Teil des Milchsammelbehälters angeformt. Die Brusthaube kann gesamthaft einstückig ausgebildet sein oder aus mehreren Teilen bestehen.

In einer anderen Ausführungsform ist mindestens eines der zwei Kopplungsteile, vorzugsweise beide Kopplungsteile, ein zum Milchsammelbehälter und Brusthaube zusätzliches Teil, wobei das zusätzliche Kopplungsteil in Richtung der Verbindung zwischen Milchsammelbehälter und Brusthaube eine Länge aufweist, welche wesentlich kleiner als die Höhe des Milchsammelbehälters und/oder der Brusthaube ist. Dadurch ist die Abpumpeinheit als Ganzes nach wie vor kompakt und platzsparend ausgebildet. Die Herstellung der einzelnen Teile ist jedoch erleichtert.

Die Relativbewegung zwischen Milchsammelbehälter und Brusthaube kann um eine einzige Schwenkachse erfolgen. Vorzugsweise ist sie um mehrere Achsen möglich. Vorzugsweise ermöglicht sie eine Schwenkbewegung im dreidimensionalen Raum.

Vorzugsweise wird die Relativbewegung durch ein Gelenk zwischen Milchsammelbehälter und Brusthaube ermöglicht. Die Abpumpeinheit kann mit einer Arretierungsvorrichtung zur Fixierung des eingestellten Winkels versehen sein. Vorzugsweise ist jedoch das Gelenk selbsthemmend ausgebildet. Der Milchsammelbehälter ist über ein Kugelgelenk mit der Brusthaube verbunden. Im Kugelgelenk ist ein Verbindungskanal vorhanden, welcher einen Innenraum der Brusthaube mit einem Innenraum des Milchsammelbehälters verbindet, um Milch von der Brusthaube in den Milchsammelbehälter zu leiten. Diese Verbindung ist einfach herzustellen und zu trennen und erlaubt einen beliebigen Schwenkwinkel im dreidimensionalen Raum. Vorteilhaft ist ferner, dass sich das Kugelgelenk, wenn auseinander genommen, einfach reinigen lässt. Es garantiert zudem Dichtheit und ermöglicht auf einfache Weise die Anordnung des Verbindungskanals im Gelenk selber. Das Kugelgelenk weist eine Gelenkkugel auf, welche an der Brusthaube angeformt oder angeordnet ist, wobei das Kugelgelenk eine Kugelaufnahme aufweist, welche in einem Deckel des Milchsammelbehälters angeformt oder angeordnet ist. Die Anordnung kann auch umgekehrt sein. So ist in einer anderen Ausfuhrungsform die Gelenkkugel an einem Deckel des Milchsammelbehälters angeformt oder angeordnet.

In einer nicht beanspruchten anderen Ausfuhrungsform ist das Gelenk ein Schwenkscharnier.

In einer weiteren nicht beanspruchten Ausfuhrungsform ist das Gelenk ein flexibler Stutzen, welcher den Milchsammelbehälter mit der Brusthaube verbindet.
Vorzugsweise ist an geeigneter Stelle in der Abpumpeinheit ein Rückschlagventil angeordnet. Dieses Rückschlagventil verhindert, dass Milch vom Sammelbehälter in die Brusthaube zurückfliessen kann. Eine "geeignete Stelle" ist eine Stelle, welche das Totvolumen in der Brusthaube möglichst minimiert und somit den beim Abpumpen mit Unterdruck zu beaufschlagenden Raum möglichst klein hält. Eine bevorzugte Stelle für die Anordnung des Ventils ist der Verbindungskanal. Das Ventil kann direkt eingesetzt, einstückig angeformt oder mittels eines Einschub- oder Einsatzelements in der Einheit, insbesondere im Kanal, befestigt sein. Vorzugsweise ist das Ventil ein Entenschnabelventil oder ein Membranventil. Andere Ventilarten lassen sich auch verwenden.

Die erfindungsgemässe Brusthaube zur Verwendung in einer oben beschriebenen Abpumpeinheit weist ein Kopplungsteil auf, welches eine Relativbewegung zwischen der Brusthaube und einem Milchsammelbehälter ermöglicht in einem Zustand, in welchem der Milchsammelbehälter an der Brusthaube befestigt ist.

Der erfindungsgemässe Milchsammelbehälter zur Verwendung in einer oben beschriebenen Abpumpeinheit ist formstabil oder annähernd formstabil ausgebildet und weist ein Kopplungsteil auf, welches eine Relativbewegung zwischen dem Milchsammelbehälter und einer Brusthaube ermöglicht in einem Zustand, in welchem der Milchsammelbehälter an der Brusthaube befestigt ist.

Die erfindungsgemässe Kopplungseinheit zur Verwendung in einer oben beschriebenen Abpumpeinheit weist ein erstes und ein zweites Kopplungsteil auf zur Verbindung einer Brusthaube und einem formstabilen oder annähernd formstabilen Milchsammelbehälter, wobei das erste und das zweite Kopplungsteil gemeinsam eine Relativbewegung zwischen der Brusthaube und dem an der Brusthaube befestigten Milchsammelbehälter ermöglichen.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine Seitenansicht einer erfindungsgemässen Abpumpeinheit gemäss einem ersten Ausfuhrungsbeispiel in einer ersten Position;
- Figur 2: die Abpumpeinheit gemäss Figur 1 in einer zweiten Position;
- Figur 3: einen Längsschnitt durch die Abpumpeinheit gemäss Figur 1;
- Figur 4: einen Längsschnitt durch eine erweiterte Ausführungsform der erfindungsgemässen Abpumpeinheit gemäss Figur 1;
- Figur 5: einen Längsschnitt durch den Behälterteil mit Deckel gemäss Figur 4;
- Figur 6: eine Ansicht des Deckels gemäss Figur 4 von oben;
- Figur 7: einen Längsschnitt durch eine erfindungsgemässe Abpumpeinheit in einer zweiten Ausführungsform;
- Figur 8: eine perspektivische Ansicht eines Milchsammelbehälters zur Verwendung in der Abpumpeinheit gemäss Figur 1;
- Figur 9: einen Längsschnitt durch eine erfindungsgemässe Abpumpeinheit in einer dritten Ausführungsform;
- Figur 10: einen Längsschnitt durch eine nicht beanspruchte weiteren Abpumpeinheit in einer vierten Ausführungsform;
- Figur 11: eine perspektivische Darstellung der Abpumpeinheit gemäss Figur 10 in einer ersten Position;
- Figur 12: eine Seitenansicht der Abpumpeinheit gemäss Figur 10 in einer zweiten Position;
- Figur 13: eine Seitenansicht durch eine nicht beanspruchte weitere Abpumpeinheit in einer fünften Ausführungsform in einer ersten Position;
- Figur 14: eine Seitenansicht der Abpumpeinheit gemäss Figur 13 in einer zweiten Position;
- Figur 15: einen Teilschnitt in einer Seitenansicht einer erweiterten nicht beanspruchten Ausfuhrungsform gemäss Figur 13 und
- Figur 16: einen Längsschnitt durch eine erfindungsgemässe Abpumpeinheit in einer sechsten Ausführungsform.

### BESCHREIBUNG BEVORZUGTER AUSFÜHRUNGSFORMEN

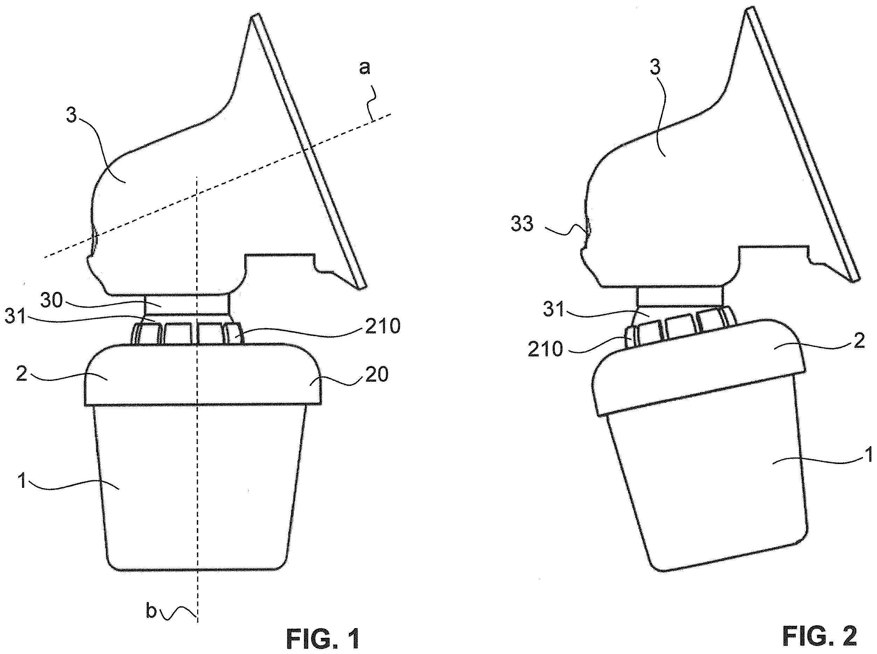

In den Figuren 1 bis 3 ist ein erstes Ausführungsbeispiel der erfindungsgemässen Abpumpeinheit dargestellt. Sie umfasst einen Milchsammelbehälter 1, 2 und eine Brusthaube 3. Die Brusthaube 3 weist eine erste Längsmittelachse a auf, der Milchsammelbehälter eine zweite Längsmittelachse b. Diese Achsen a, b stehen in einem Winkel zueinander.
Der Milchsammelbehälter 1 weist einen Behälterteil 1 mit einem Innenraum zur Aufnahme der abgepumpten Milch und einen diesen Behälterteil 1 verschliessenden Deckel 2 auf. Mindestens der Behälterteil 1, vorzugsweise auch der Deckel 2, ist formstabil oder annähernd formstabil ausgebildet. Vorzugsweise sind sie relativ steif ausgebildet. Sie bestehen üblicherweise aus einem Kunststoff. Der Deckel 2 lässt sich in einer einfachen Ausfuhrungsform lösbar auf den Behälterteil 1 aufstecken. Der Behälterteil 1 weist hierfür eine oder mehrere Rückhalterippen 10 auf, welche seinen oberen Umfang umlaufen und in den Deckel 2, vorzugsweise in eine entsprechende umlaufende Nut, eingreifen. Ist der Behälter rund ausgebildet, so lässt sich alternativ auch ein Drehverschluss verwenden. Klemmen oder andere Befestigungsmittel sind ebenfalls möglich.
Der Behälterteil 1 ist vorzugsweise selbststehend ausgebildet. Er weist hierfür einen stabilen Bodenbereich oder vorstehende Fussnoppen auf.
Der Deckel 2 weist einen Deckelkörper 20 mit einer nach oben gerichteten Oberfläche auf. In dieser Oberfläche ist eine Gelenkkugelaufnahme 21 vorhanden. Die Gelenkkugelaufnahme 21 ist teilkugelförmig ausgebildet, wobei federnde Klammern 210 eine runde Öffnung umgeben. Diese runde Öffnung führt in den Innenraum des Behälterteils 1.

Die Brusthaube 3 ist ebenfalls vorzugsweise aus Kunststoff gefertigt. Sie weist einen sich nach aussen erweiternden Trichter 32 zur Aufnahme der Mutterbrust auf. Der Trichter 32 endet in einem röhrenförmigen Abschnitt 39 mit einem Vakuumanschluss 33 zur Verbindung mit einer Vakuumpumpe bzw. mit einem zur Vakuumpumpe führenden Schlauch. Trichter 32 und röhrenförmiger Abschnitt 39 sind beide relativ klein gehalten. Anstelle der dargestellten klassischen Trichterform lassen sich auch andere Formen von Brusthauben einsetzen. Dies gilt auch für die übrigen in diesem Text beschriebenen Ausführungsbeispiele. Die Brusthaube 3 ist vorzugsweise, wie hier dargestellt, einstückig ausgebildet. Sie kann jedoch auch mehrstückig ausgebildet sein. Die Brusthaube 3 ist vorzugsweise aus einem steifen oder annähernd steifen Material gefertigt. Sie kann jedoch, insbesondere im Bereich des Trichters 32, auch aus einem relativ weichen, sich der Mutterbrust in der Form anpassenden Material gefertigt sein. Mindestens der röhrenförmige Teil 39 ist jedoch vorzugsweise steif oder zumindest formstabil ausgebildet. Auch diese Varianten sind für die übrigen Ausführungsbeispiele möglich.

Der untere Bereich des röhrenförmigen Abschnitts 39 der Brusthaube 3 geht in einen steifen Hals 30 über, an welchem eine Gelenkkugel 31 angeformt ist. Diese Gelenkkugel 31 ist lösbar in der Gelenkkugelaufnahme 21 gehalten. Sie lässt sich innerhalb der Aufnahme drehen, wobei das Gelenk vorzugsweise selbsthemmend ausgebildet ist, so dass die Kugel 31 und somit die Brusthaube 3 ohne weitere Fixierungsmittel in einer von der Mutter gewählten Position relativ zum Behälter 1, 2 verbleibt. Diese Hemmung erfolgt vorzugsweise mittels der federnden Klammern 210.

Ein Verbindungskanal 34 verläuft durch den Hals 30 und die Gelenkkugel 31 und verbindet den Innenraum 390 der Brusthaube 3 mit dem Innenraum 11 des Behälterteils 1. Im Verbindungskanal 34 ist ein Rückschlagventil, hier ein Entenschnabelventil 4 angeordnet. Es liegt in diesem Beispiel auf einem Absatz des Verbindungskanals 34 auf. Dieser Absatz ist am oberen Ende des Verbindungskanals 34 ausgebildet, d.h. beim Übergang zum röhrenförmigen Abschnitt 39. Dadurch lässt sich das Ventil 4 über den Trichter 32 der Brusthaube 3 an diese Position einführen.

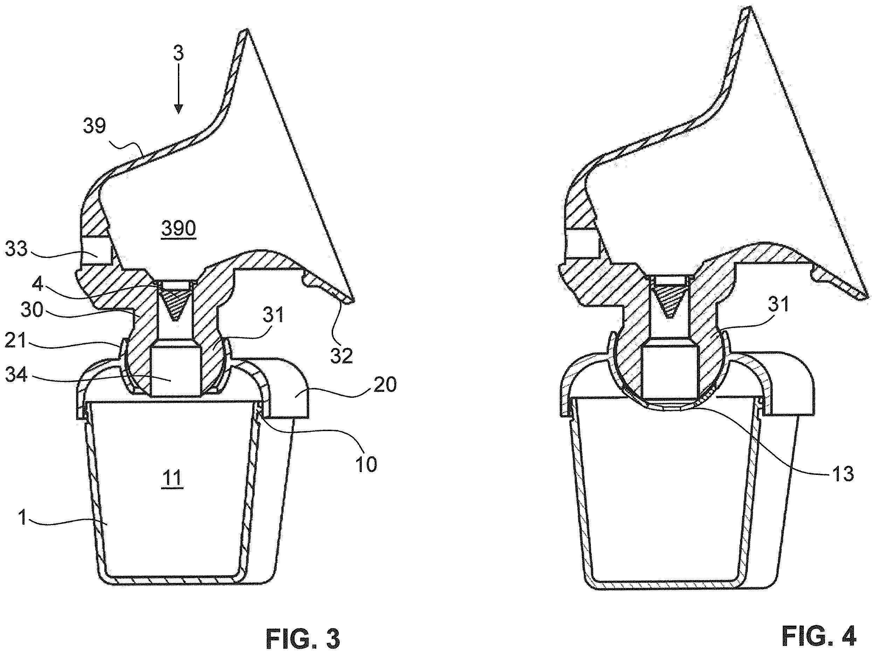

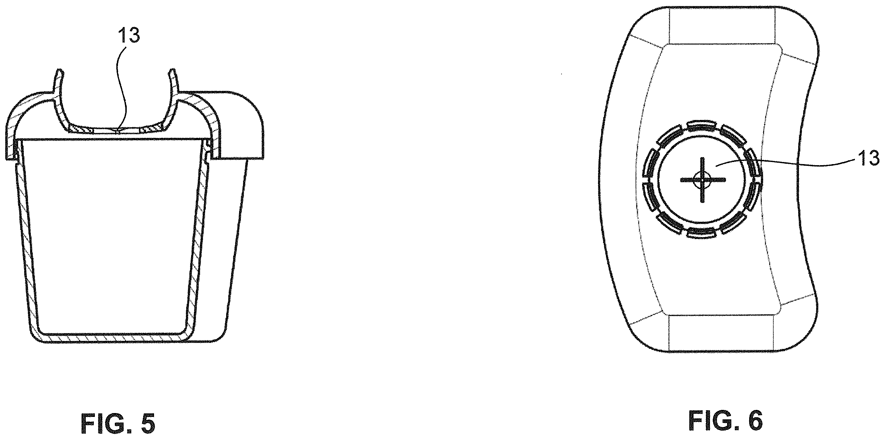

In den Figuren 4 bis 6 ist eine erweiterte Ausführungsform des oben beschriebenen Beispiels dargestellt. Der Deckelkörper 2 ist in dieser Ausfuhrungsform mit einem reversiblen Verschluss 13 ausgestattet. Dieser Verschluss 13 öffnet sich beim Einsetzen der Gelenkkugel 31. Der Verschluss 13 kann als federnde Segmente, z.B. wie hier dargestellt als Kreuzschlitzmembran, ausgebildet sein. Somit kann der Behälter zu Lagerungszwecken mindestens annähernd dicht aufbewahrt werden, ohne dass ein zusätzlicher Deckel notwendig ist.

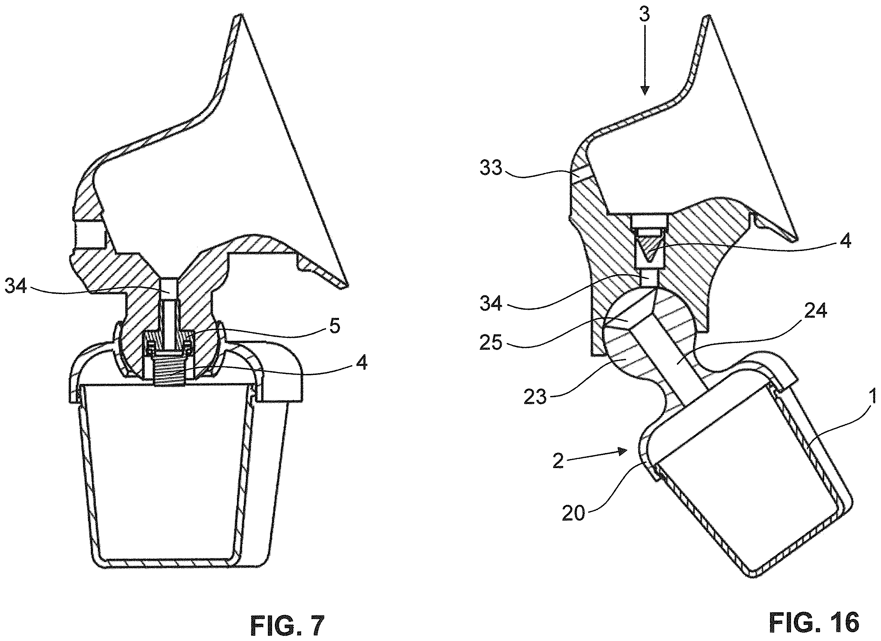

In der Ausfuhrungsform gemäss Figur 7 ist das Ventil 4 in einem Einschubelement 5 gehalten, welches sich von unten, d.h. vom anderen freien Ende des Verbindungskanals 34 einführen lässt. Der Kanal 34 ist entsprechend mit einem Anschlag versehen, auf welchem eine umlaufende Schulter des Einschubelements 5 anliegt. Das Einschubelement 5 ist vorzugsweise ebenfalls aus Kunststoff gefertigt. Das Ventil 4 kann, wie hier dargestellt, als separates Teil ausgebildet sein oder einstückig mit dem Einschubelement 5 ausgebildet sein.

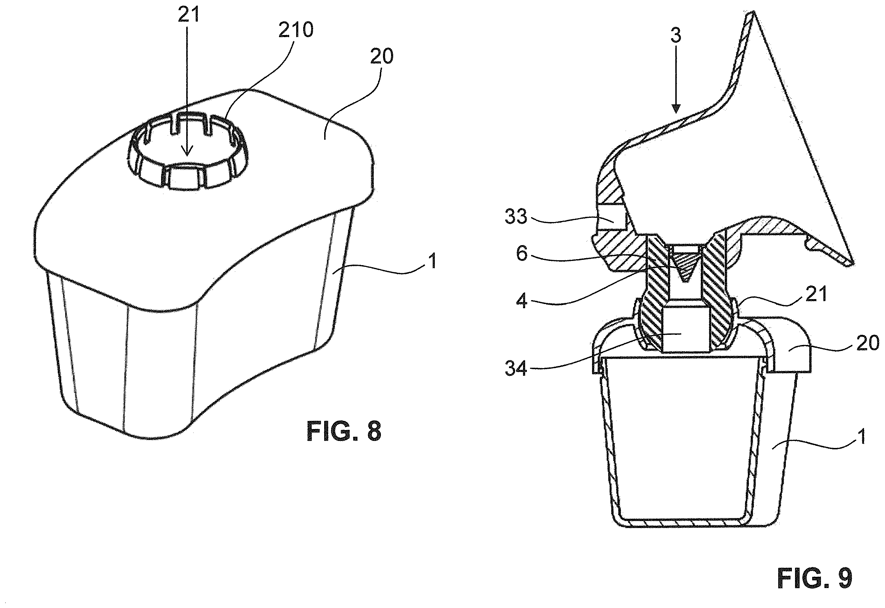

Figur 8 zeigt eine bevorzugte Form eines Milchsammelbehälters 1, 2, welcher sich in der erfindungsgemässen Abpumpeinheit verwenden lässt. Der Behälterteil 1 weist vorzugsweise einen nierenförmigen Querschnitt auf, so dass er eine nach innen gebogene Wand und eine gegenüberliegende nach aussen gebogene Wand aufweist. Der Deckel 2 ist analog ausgebildet. Die nach innen gebogene Wand lässt sich an den Körper der Mutter, z.B. an die Mutterbrust anlegen, so dass der Behälter sehr nahe an den Körper gebracht werden kann. Dieser Behälter lässt sich in allen hier beschriebenen Ausführungsformen der erfindungsgemässen Abpumpeinheit verwenden. Er lässt sich auch in anderen Abpumpeinheiten einsetzen, um eine möglichst platzsparende Anordnung an der Brust zu ermöglichen. Vorteilhaft ist ferner, dass sich mehrere Behälterteile 1 sowohl in vertikaler Richtung ineinander stapeln lässt wie auch in horizontaler Richtung ohne Zwischenräume nebeneinander anordnen lassen. Dadurch lassen sie sich äusserst platzsparend lagern.

In der Ausführungsform gemäss Figur 9 ist ein Adapterteil 6 zwischen Brusthaube 3 und Milchsammelbehälter 1, 2 angeordnet. Die Brusthaube 3 ist auf dem Adapterteil 6 aufgesteckt. Sie kann lösbar mit ihm verbunden sein, damit die einzelnen Teile getrennt voneinander gereinigt und wieder zusammengesetzt werden können. Sie kann jedoch auch nicht zerstörungsfrei fest mit ihm verbunden sein. Dies ist insbesondere bei Brusthauben 3, welche für den Einmalgebrauch oder für einen kurzzeitigen Mehrfachgebrauch bestimmt sind, vorteilhaft.
Das Adapterteil 6 bildet den Hals der Brusthaube 3 und weist an seinem freien Ende die Gelenkkugel auf. Eine Durchgangsöffnung bildet den Verbindungskanal 34. Auch diese Ausfiihrungsform weist vorzugsweise ein Rückschlagventil 4 auf. Dieses ist hier wiederum am oberen Ende angeordnet. Es kann jedoch auch mit dem Einschubelement 5 gemäss Figur 7 oder einer anderen Ventilanordnung versehen sein.

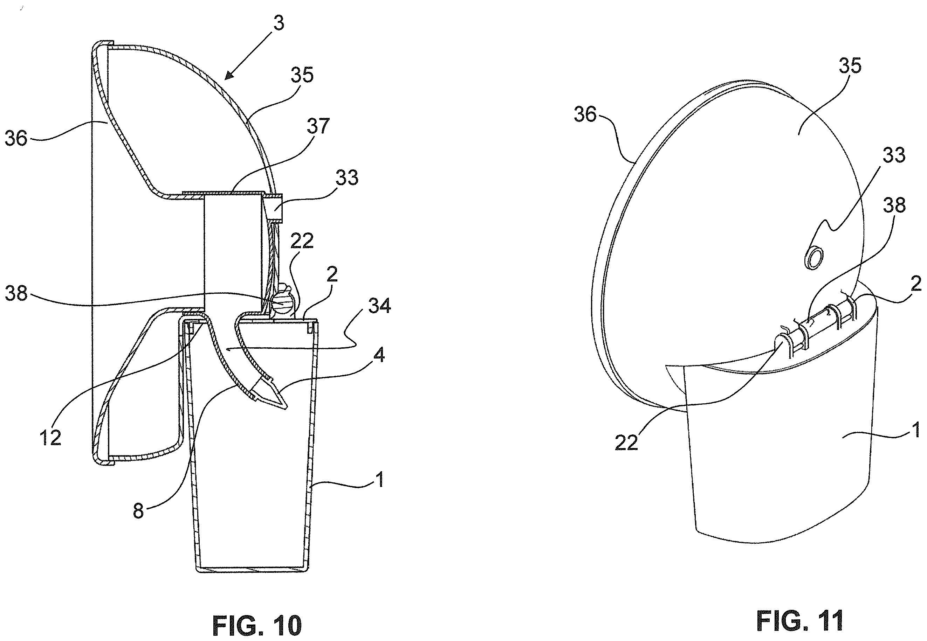

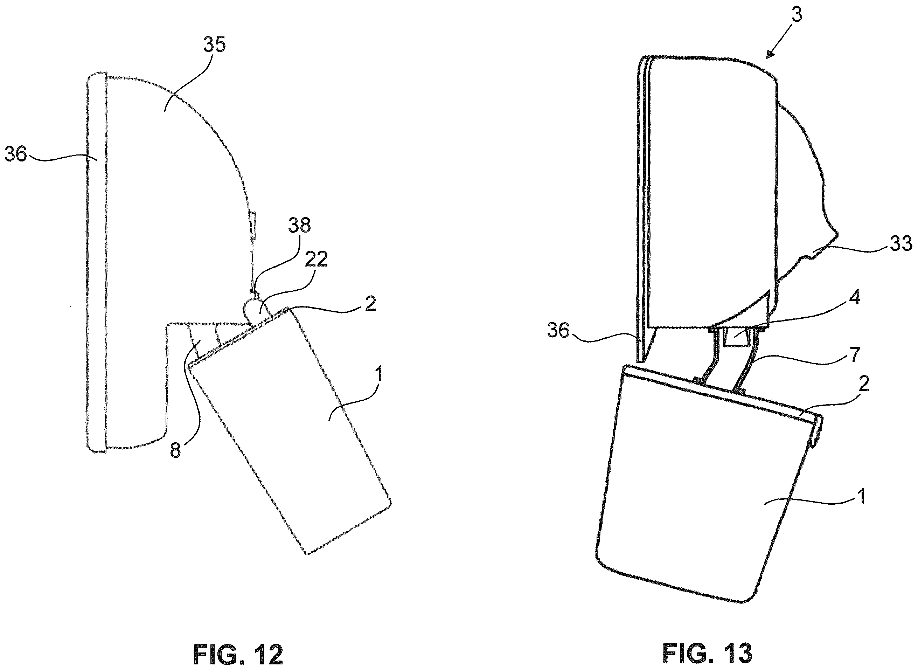

In den Figuren 10 bis 12 ist ein nicht beanspruchtes weiteres Ausführungsbeispiel dargestellt. In den bisherigen Beispielen wurde die Milch durch das Gelenk geleitet. In diesem Beispiel ist das Gelenk getrennt vom Verbindungskanal 34 angeordnet. Das Gelenk ist durch ein Scharniergelenk gebildet. An der Brusthaube 3 ist ein Scharnierstift 38 befestigt, vorzugsweise einstückig angeformt, welcher in einem Scharnierträger 22 schwenkbar gehalten ist. Der Scharnierträger 22 ist wiederum auf dem Deckel 2 des Milchsammelbehälters befestigt, vorzugsweise ebenfalls einstückig mit diesem ausgebildet. Der Deckel 2 ist vorzugsweise, wie in diesem Beispiel dargestellt, plan ausgebildet. Er weist beabstandet zum Scharnier eine Durchlassöffnung 12 auf, durch welche eine Milchleitung 8 ragt. Die Milchleitung 8 ist fest mit der Brusthaube 3 verbunden. Sie bildet den Durchgangskanal 34. Die Milchleitung 8 ist vorzugsweise ein steifer oder flexibler Schlauch. Auch hier ist vorzugsweise wiederum ein Rückschlagventil 4 vorhanden. Es kann beispielsweise am Anfang oder wie hier dargestellt am Ende der Milchleitung 8 an dieser befestigt sein.
Es lässt sich auch hier dieselbe Brusthaube 3 verwenden wie im ersten Beispiel. Die dargestellte Variante ist jedoch in dieser Kombination äusserst platzsparend und kompakt. Sie weist eine halbkreisförmige, vorzugsweise steife Brusthaubenschale 35 auf, in welcher ein Trichtereinsatz 36 gehalten ist. Er weist einen umlaufenden Rand auf, welcher über den umlaufenden Rand der Brusthaubenschale 35 gestülpt ist. Das schmale Ende des Trichtereinsatzes 36 ist in einer vorzugsweise steifen Trichteraufnahme 37 gehalten. Diese Trichteraufnahme 37 ist in der Brusthaubenschale 35 befestigt, wobei sie einen Stutzen aufweist, welcher die Brusthaubenschale 35 nach aussen durchdringt und den Vakuumanschluss 33 bildet. Vorzugsweise ist, wie hier dargestellt, die Milchleitung 8 einstückig mit der Trichteraufnahme 37 ausgebildet oder an ihr befestigt.
Wie in der Zusammenschau der Figuren 10 und 12 erkennbar ist, lässt sich nun die Brusthaube 3 relativ zum Behälter 1, 2 um eine Schwenkachse schwenken. Die Durchlassöffnung 12 ist dabei so gross bemessen, dass die Milchleitung 8 darin genügend Spielraum hat, um die Bewegung mitzumachen, ohne geknickt zu werden. Es ist nicht notwendig, dass der Behälter 1, 2 in diesem Bereich vollständig verschlossen ist.

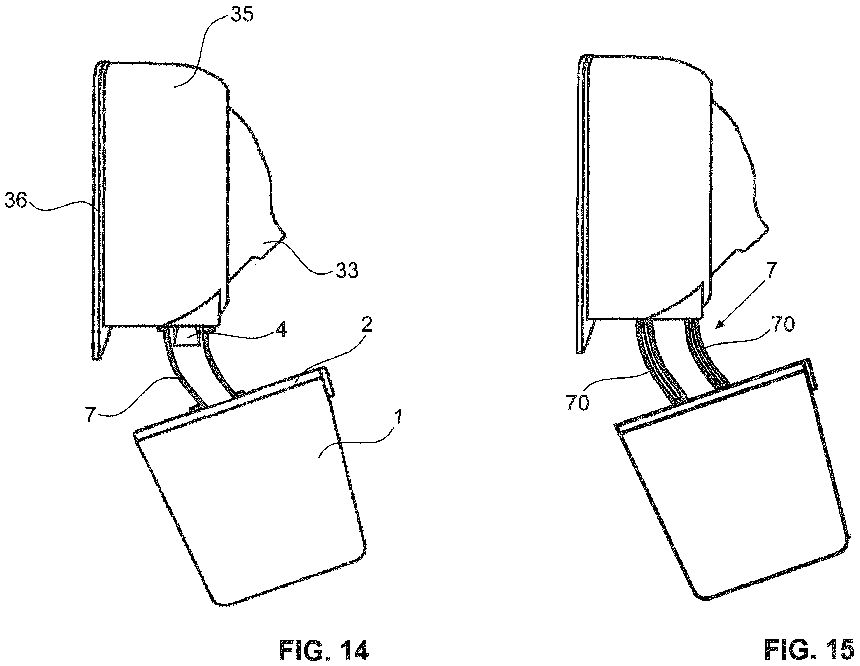

In den Figuren 14 und 16 ist eine nicht beanspruchte weitere Ausfuhrungsform dargestellt. Hier ist das Gelenk durch einen flexiblen Stutzen 7 gebildet. Der Stutzen 7 weist vorzugsweise Längen im Bereich von 20 bis 50 mm auf. Der Stutzen ist vorzugsweise durch einen Schlauch, vorzugsweise einen Breitbandschlauch, d.h. einem flachen Schlauch gebildet. Vorzugsweise weist er auf seiner Innenseite Strukturen, wie beispielsweise Längsrippen auf, damit er nicht knickt und/oder sich der Querschnitt nicht verschliessen kann. Er ist vorzugsweise steif genug ausgebildet, um eine eingestellte Position selbsttätig zu halten, so dass der Behälter 1,2 die Brusthaube 3 innerhalb einer bestimmten Bandbreite von Schwenkwinkeln in unterschiedlichen Schwenkwinkeln und ohne äusseren Kraftaufwand tragen kann.

In einer nicht beanspruchten Ausführungsform, welche in Figur 15 dargestellt ist, weist der Schlauch bzw. Stutzen 7 Verstärkungselemente auf. Vorzugsweise sind dies ein oder mehrere Drähte 70, welche in oder an der Wandung des Stutzens 7 angeordnet sind.
Der Stutzen 7 ist einerseits an der Brusthaube 3 und andererseits am Deckel 2 des Milchsammelbehälters befestigt. Er kann auf beiden Seiten lösbar befestigt sein oder an einer oder beiden Seiten fest und nicht zerstörungsfrei lösbar mit diesen Teilen verbunden sein. Vorzugsweise ist er eingesteckt und weist entsprechende Rückhalterippen und/odernuten auf. Der Stutzen 7 ermöglicht vorzugsweise eine Schwenkung in einer Hauptrichtung unter einer leichten Neigung in anderen Richtungen.
Auch in diesem Beispiel ist vorzugsweise ein Rückschlagventil 4 vorhanden. Hier ist es wiederum in der Brusthaube 3 angeordnet.

In der Ausfuhrungsform gemäss Figur 16 ist das Gelenk wiederum ein Kugelgelenk. Hier ist jedoch am Deckel 2 des Milchsammelbehälters eine Gelenkkugel 23 angeordnet, insbesondere einstückig mit ihm gefertigt. Die Brusthaube 3 weist eine entsprechende Gelenkkugelaufnahme auf. Der Verbindungskanal 34 der Brusthaube 3 geht in einen Verbindungskanal 24 in der Gelenkkugel 23 über. Damit diese Verbindung auch bei relativ grossen Schwenkwinkeln offen bleibt, ist das obere Ende des deckelseitigen Verbindungskanals 24 mit einem sich erweiternden Einlasstrichter 25 versehen.

Die erfindungsgemässe Abpumpeinheit lässt sich kompakt und platzsparend ausbilden und eignet sich insbesondere in einer "hands-free" Anordnung.

**BEZUGSZEICHENLISTE**

| | | | |
|---|---|---|---|
| 1 | Behälterteil | 35 | Brusthaubenschale |
| 10 | Rückhalterippe | 36 | Trichtereinsatz |
| 11 | Innenraum | 37 | Trichteraufnahme |
| 12 | Durchlassöffnung | 38 | Scharnierstift |
| 13 | Verschluss | 39 | röhrenförmiger Abschnitt |
| | | 390 | Innenraum |
| 2 | Deckel | | |
| 20 | Deckelkörper | 4 | Ventil |
| 21 | Kugelaufnahme | | |
| 210 | Klammer | 5 | Einschubelement |
| 22 | Scharnierträger | | |
| 23 | Gelenkkugel | 6 | Adapterteil |
| 24 | Verbindungskanal | | |
| 25 | Einlasstrichter | 7 | Stutzen |
| | | 70 | Draht |
| 3 | Brusthaube | | |
| 30 | Hals | 8 | Milchleitung |
| 31 | Gelenkkugel | | |
| 32 | Trichter | a | erste Längsmittelachse |
| 33 | Vakuumanschluss | b | zweite Längsmittelachse |
| 34 | Verbindungskanal | | |

## Patentansprüche

1. Abpumpeinheit zur Verwendung in einer Vorrichtung zum Abpumpen von menschlicher Muttermilch, wobei die Abpumpeinheit eine Brusthaube (3) zur Anlage an eine Mutterbrust und einen formstabilen oder annähernd formstabilen Milchsammelbehälter (1, 2) zur Aufnahme der abgepumpten Muttermilch aufweist, wobei die Brusthaube (3) eine erste Längsmittelachse (a) und der Milchsammelbehälter (1, 2) eine zweite Längsmittelachse (b) aufweist, wobei der Milchsammelbehälter (1, 2) lösbar an der Brusthaube (3) befestigbar ist und wobei die erste Längsmittelachse (a) im befestigten Zustand des Milchsammelbehälters (1, 2) in einem Winkel zur zweiten Längsmittelachse (b) angeordnet ist, wobei der Milchsammelbehälter (1, 2) im befestigten Zustand relativ zur Brusthaube (3) bewegbar ist, wodurch der Winkel zwischen der ersten Längsmittelachse (a) und zweiten Längsmittelachse (b) veränderbar ist, wobei ein erstes und ein zweites Kopplungsteil (31, 21; 23) vorhanden sind, welche gemeinsam die Relativbewegung ermöglichen, wobei das erste Kopplungsteil (31) an der Brusthaube (3) und das zweite Kopplungsteil (21, 23) am Milchsammelbehälter (1, 2) angeordnet ist, wobei das erste Kopplungsteil (31) mit dem zweiten Kopplungsteil (21, 23) ein Kugelgelenk bildet, welches den Milchsammelbehälter (1, 2) mit der Brusthaube (3) verbindet, wobei im Kugelgelenk ein Verbindungskanal (24, 34) vorhanden ist, welcher einen Innenraum (390) der Brusthaube (3) mit einem Innenraum (11) des Milchsammelbehälters (1) verbindet, um Milch von der Brusthaube (3) in den Milchsammelbehälter (1, 2) zu leiten, **dadurch gekennzeichnet, dass** eine Position der Brusthaube (3) existiert, in welcher sich dieser Verbindungskanal (24, 34) in Richtung der zweiten Längsmittelachse (b) erstreckt, und dass entweder
a) das Kugelgelenk eine Gelenkkugel (31) aufweist, welche an der Brusthaube (3) angeformt oder angeordnet ist, wobei das Kugelgelenk eine Kugelaufnahme (21) aufweist, welche in einem Deckel (2) des Milchsammelbehälters (1, 2) angeformt oder angeordnet ist, oder dass
b) das Kugelgelenk eine Gelenkkugel (23) aufweist, welche an einem Deckel (2) des Milchsammelbehälters (1, 2) angeformt oder angeordnet ist.

2. Abpumpeinheit nach Anspruch 1, wobei der Milchsammelbehälter ein Behälterteil (1) und den diesen Behälterteil (1) verschliessenden Deckel (2) aufweist.

3. Abpumpeinheit nach einem der Ansprüche 1 oder 2, wobei das erste Kopplungsteil (31) einstückig an einem Teil der Brusthaube (3) und/oder das zweite Kopplungsteil (21, 23) einstückig an einem Teil des Milchsammelbehälters (1, 2) angeformt ist.

4. Abpumpeinheit nach einem der Ansprüche 1 oder 2, wobei mindestens eines der zwei Kopplungsteile (6) ein zum Milchsammelbehälter (1, 2) und Brusthaube (3) zusätzliches Teil ist, wobei das zusätzliche Kopplungsteil (6) in Richtung der Verbindung zwischen Milchsammelbehälter (1, 2) und Brusthaube (3) eine Länge aufweist, welche wesentlich kleiner als die Höhe des Milchsammelbehälters (1, 2) und/oder der Brusthaube (3) ist.

5. Abpumpeinheit nach einem der Ansprüche 1 bis 4, wobei der Milchsammelbehälter (1,2) einen nierenförmigen Querschnitt aufweist.

6. Brusthaube (3) einer Abpumpeinheit nach einem der Ansprüche 1 bis 5, wobei die Brusthaube (3) eine erste Längsmittelachse (a) definiert und ein erstes Kopplungsteil (31) aufweist, welches eine Relativbewegung zwischen der Brusthaube (3) und einem Milchsammelbehälter (1, 2) ermöglicht in einem Zustand, in welchem der Milchsammelbehälter (1, 2) an der Brusthaube (3) befestigt ist, wobei der Milchsammelbehälter (1, 2) eine zweite Längsmittelachse (b) definiert, wobei das erste Kopplungsteil (31) mit einem zweiten Kopplungsteil (21, 23) des Milchsammelbehälters (1, 2) verbindbar ist, wobei die zwei Kopplungsteile gemeinsam die Relativbewegung ermöglichen, wobei der Winkel zwischen der ersten Längsmittelachse (a) und der zweiten Längsmittelachse (b) im befestigen Zustand des Milchsammelbehälters (1, 2) veränderbar ist, , wobei das erste Kopplungsteil (31) mit dem zweiten Kopplungsteil (21, 23) ein Kugelgelenk bildet, wobei im Kugelgelenk ein Verbindungskanal (24, 34) vorhanden ist, welcher einen Innenraum (390) der Brusthaube (3) mit einem Innenraum (11) des Milchsammelbehälters (1) verbindet, um Milch von der Brusthaube (3) in den Milchsammelbehälter (1, 2) zu leiten, wobei das erste Kopplungsteil eine Gelenkkugel (31) oder eine Kugelaufnahme (21) des Kugelgelenks aufweist und das zweite Kopplungsteil (21, 23) eine entsprechende Kugelaufnahme (21) oder eine Gelenkkugel (31) des Kugelgelenks aufweist und wobei eine Position der Brusthaube (3) existiert, in welcher sich dieser Verbindungskanal (24, 34) in Richtung der zweiten Längsmittelachse (b) erstreckt, **dadurch gekennzeichnet, dass** eine Position der Brusthaube (3) existiert, in welcher sich der Verbindungskanal (24, 34) in eine Richtung erstreckt, die mit der ersten Längsmittelachse (a) einen Winkel bildet.

7. Milchsammelbehälter (1, 2) zur Verwendung in einer Abpumpeinheit nach einem der Ansprüche 1 bis 5, wobei der Milchsammelbehälter (1, 2) formstabil oder annähernd formstabil ausgebildet ist und wobei er ein zweites Kopplungsteil (21, 23) aufweist, welches eine Relativbewegung zwischen dem Milchsammelbehälter (1, 2) und einer Brusthaube (3) ermöglicht in einem Zustand, in welchem der Milchsammelbehälter (1, 2) an der Brusthaube (3) befestigt ist, wobei der Milchsammelbehälter (1, 2) eine zweite Längsmittelachse (b) definiert, wobei das zweite Kopplungsteil (21, 23) mit einem ersten Kopplungsteil (31) der Brusthaube (3) verbindbar ist, wobei die zwei Kopplungsteile gemeinsam die Relativbewegung ermöglichen, wobei der Winkel zwischen der ersten Längsmittelachse (a) und der zweiten Längsmittelachse (b) im befestigen Zustand des Milchsammelbehälters (1, 2) veränderbar ist, wobei das erste Kopplungsteil (31) mit dem zweiten Kopplungsteil (21, 23) ein Kugelgelenk bildet, wobei im Kugelgelenk ein Verbindungskanal (24, 34) vorhanden ist, welcher einen Innenraum (390) der Brusthaube (3) mit einem Innenraum (11) des Milchsammelbehälters (1) verbindet, um Milch von der Brusthaube (3) in den Milchsammelbehälter (1, 2) zu leiten, wobei das erste Kopplungsteil eine Gelenkkugel (31) oder eine Kugelaufnahme (21) des Kugelgelenks aufweist und das zweite Kopplungsteil (21, 23) eine entsprechende Kugelaufnahme (21) oder eine Gelenkkugel (31) des Kugelgelenks aufweist und wobei eine Position der Brusthaube (3) existiert, in welcher sich der Verbindungskanal (24, 34) in Richtung der zweiten Längsmittelachse (b) erstreckt.

8. Kopplungseinheit (31, 21; 23) zur Verwendung in einer Abpumpeinheit gemäss einem der Ansprüche 1 bis 5, wobei die Kopplungseinheit (31, 21; 23) ein erstes und ein zweites Kopplungsteil aufweist zur Verbindung einer Brusthaube (3) mit einer ersten Längsmittelachse (a) und einem formstabilen oder annähernd formstabilen Milchsammelbehälter (1, 2) mit einer zweiten Längsmittelachse (b), wobei das erste und das zweite Kopplungsteil gemeinsam eine Relativbewegung zwischen der Brusthaube (3) und dem an der Brusthaube (3) befestigten Milchsammelbehälter (1, 2) ermöglichen, , wobei der Winkel zwischen der ersten Längsmittelachse (a) und der zweiten Längsmittelachse (b) im befestigen Zustand des Milchsammelbehälters (1, 2) an der Brusthaube (3) veränderbar ist, wobei das erste Kopplungsteil (31) mit dem zweiten Kopplungsteil (21, 23) ein Kugelgelenk bildet, wobei im Kugelgelenk ein Verbindungskanal (24, 34) vorhanden ist, welcher einen Innenraum (390) der Brusthaube (3) mit einem Innenraum (11) des Milchsammelbehälters (1) verbindet, um Milch von der Brusthaube (3) in den Milchsammelbehälter (1, 2) zu leiten und wobei das erste Kopplungsteil eine Gelenkkugel (31) oder eine Kugelaufnahme (21) des Kugelgelenks aufweist und das zweite Kopplungsteil (21, 23) eine entsprechende Kugelaufnahme (21) oder eine Gelenkkugel (31) des Kugelgelenks aufweist und wobei eine Position der Brusthaube (3) existiert, in welcher sich dieser Verbindungskanal (24, 34) in Richtung der zweiten Längsmittelachse (b) erstreckt.

## Claims

1. A pumping unit for use in a device for expressing human breast milk wherein the pumping unit comprises a breast shield (3) for resting against a mother's breast and a dimensionally stable or approximately dimensionally stable milk collection container (1, 2) for receiving the expressed milk, wherein the breast shield (3) comprises a first longitudinal centre axis (a), and the milk collection container (1, 2) comprises a second longitudinal centre axis (b), wherein the milk collection container (1, 2) is detachably attachable to the breast shield (3), and wherein in the attached state of the milk collection container (1,2) the first longitudinal centre axis (a) is arranged at an angle to the second longitudinal centre axis (b), wherein in the attached state the milk collection container (1, 2) is movable relative to the breast shield (3), and consequently the angle between the first longitudinal centre axis (a) and the second longitudinal centre axis (b) is variable, wherein there is a first and a second coupling part (31, 21; 23), which parts together enable the relative movement, wherein the first coupling part (31) is arranged on the breast shield (3) and the second coupling part (21, 23) is arranged on the milk collection container (1, 2), wherein the first coupling part (31) together with the second coupling part (21, 23) form a ball joint which connects the milk collection container (1,2) with the breast shield (3), wherein within the ball joint a connection channel (4, 34) a connection channel (34, 24) is present, which connects an interior space (390) of the breast shield (3) with an interior space (11) of the milk collection container (1) in order to lead milk from the breast shield (3) to the milk collection container (1, 2), **characterized in that** there exists a position of the breast shield (3) in which the connection channel (34, 24) extends in direction of the second longitudinal centre axis (b) and that either
a) the ball joint comprises a joint ball (31) that is formed to or arranged on the breast shield (3), wherein the ball joint comprises a ball holder (21) that is formed to or arranged on a lid (2) of the milk collection container (1, 2) or that
b) the ball joint comprises a joint ball (23) that is formed to or arranged on a lid (2) of the milk collection container (1, 2).

2. The pumping unit according to claim 1, wherein the milk collection container comprises a container part (1) and the lid (2) closing off this container part (1).

3. The pumping unit according to any one of claims 1 or 2, wherein the first coupling part (31) is formed in a single piece to a part of the breast shield (3) and/or the second coupling part (21, 23) is formed in a single piece to a part of the milk collection container (1, 2).

4. The pumping unit according to any one of claims 1 or 2, wherein at least one of the two coupling parts (6) is a part that is additional to the milk collection container (1, 2) and the breast shield (3), wherein the additional coupling part (6) in the direction of the connection between the milk collection container (1, 2) and the breast shield (3) comprises a length that is significantly shorter than the height of the milk collection container (1, 2) and/or of the breast shield (3).

5. The pumping unit according to any one of claims 1 to 4, wherein the milk collection container (1, 2) comprises a kidney-shaped cross-section.

6. A breast shield (3) of a pumping unit according to any one of claims 1 to 5, wherein the breast shield (3) defines a first longitudinal centre axis (a) and comprises a first coupling part (31) that allows relative movement between the breast shield (3) and a milk collection container (1, 2) in a state in which the milk collection container (1, 2) is attached to the breast shield (3), wherein the milk collection container (1, 2) defines a second longitudinal centre axis (b), wherein the first coupling part (31) is connectable to a second coupling part (21, 23) of the milk collection container (1, 2), wherein the two coupling parts together enable the relative movement, wherein the angle between the first longitudinal centre axis (a) and the second longitudinal centre axis (b) is changeable in the attached state of the milk collection container (1, 2), wherein the first coupling part (31) together with the second coupling part (21, 23) form a ball joint, wherein within the ball joint a connection channel (24, 34) is present, which connects an interior space (390) of the breast shield (3) with an interior space (11) of the milk collection container (1) in order to lead milk from the breast shield (3) to the milk collection container (1, 2), wherein the first coupling part comprises a joint ball (31) or a ball holder (21) of the ball joint and the second coupling part (21, 23) comprises a corresponding ball holder (21) or a joint ball (31) of the ball joint and wherein there exists a position of the breast shield (3) in which the connection channel (34, 24) extends in direction of the second longitudinal centre axis (b), **characterized in that** there exists a position of the breast shield (3) in which the connection channel (34, 24) extends in a direction forming an angle with the first longitudinal centre axis (a).

7. A milk collection container (1,2) for use in a pumping unit according to any one of claims 1 to 5, wherein the milk collection container (1, 2) is designed to be dimensionally stable or approximately dimensionally stable and wherein it comprises a second coupling part (21, 23) that allows relative movement between the milk collection container (1, 2) and a breast shield (3) in a state in which the milk collection container (1, 2) is attached to the breast shield (3), wherein the milk collection container (1, 2) defines a second longitudinal centre axis (b), wherein the second coupling part (21, 23) is connectable to a first coupling part (31) of the breast shield (3), wherein the two coupling parts together enable the relative movement, wherein the angle between the first longitudinal centre axis (a) and the second longitudinal centre axis (b) is changeable in the attached state of the milk collection container (1, 2), wherein the first coupling part (31) together with the second coupling part (21, 23) form a ball joint, wherein within the ball joint a connection channel (24, 34) is present, which connects an interior space (390) of the breast shield (3) with an interior space (11) of the milk collection container (1) in order to lead milk from the breast shield (3) to the milk collection container (1, 2), wherein the first coupling part comprises a joint ball (31) or a ball holder (21) of the ball joint and the second coupling part (21, 23) comprises a corresponding ball holder (21) or a joint ball (31) of the ball joint and wherein there exists a position of the breast shield (3) in which the connection channel (34, 24) extends in direction of the second longitudinal centre axis (b).

8. A coupling unit (31, 21; 23) for use in a pumping unit according to any one of claims 1 to 5, wherein the coupling unit (31, 21; 23) comprises a first coupling part and a second coupling part for connecting a breast shield (3) with a first longitudinal centre axis (a) and a dimensionally stable or approximately dimensionally stable milk collection container (1, 2) with a second longitudinal centre axis (b), wherein the first and the second coupling part together allow relative movement between the breast shield (3) and the milk collection container (1, 2) attached to the breast shield (3), wherein the angle between the first axis (a) and the second longitudinal centre axis (b) is changeable in the attached state of the milk collection container (1, 2), wherein the first coupling part (31) together with the second coupling part (21, 23) form a ball joint, wherein within the ball joint a connection channel (24, 34) is present, which connects an interior space (390) of the breast shield (3) with an interior space (11) of the milk collection container (1) in order to lead milk from the breast shield (3) to the milk collection container (1, 2), wherein the first coupling part comprises a joint ball (31) or a ball holder (21) of the ball joint and the second coupling part (21, 23) comprises a corresponding ball holder (21) or a joint ball (31) of the ball joint and wherein there exists a position of the breast shield (3) in which the connection channel (34, 24) extends in direction of the second longitudinal centre axis (b).

## Revendications

1. Unité de tirage de lait pour l'utilisation dans un dispositif de tirage de lait maternel humain, l'unité de tirage de lait présentant une téterelle (3) destinée à être appliquée contre un sein maternel et un récipient de collecte de lait (1, 2) de forme stable ou approximativement stable pour recevoir le lait maternel tiré, la téterelle (3) présentant un premier axe médian longitudinal (a) et le récipient de collecte de lait (1, 2) présentant un deuxième axe médian longitudinal (b), le récipient de collecte de lait (1, 2) pouvant être fixé de manière amovible à la téterelle (3) et le premier axe médian longitudinal (a) étant disposé dans l'état fixé du récipient de collecte de lait (1, 2) suivant un certain angle par rapport au deuxième axe médian longitudinal (b), le récipient de collecte de lait (1, 2), pouvant être déplacé par rapport à la téterelle (3) dans l'état fixé, de sorte que l'angle entre le premier axe médian longitudinal (a) et le deuxième axe médian longitudinal (b) puisse être modifié, une première et une deuxième partie d'accouplement (31, 21 ; 23) étant prévues, lesquelles permettent ensemble le mouvement relatif, la première partie d'accouplement (31) étant disposée au niveau de la téterelle (3) et la deuxième partie d'accouplement (21, 23) étant disposée au niveau du récipient de collecte de lait (1, 2), la première partie d'accouplement (31) formant avec la deuxième partie d'accouplement (21, 23) une articulation à rotule qui relie le récipient de collecte de lait (1, 2) à la téterelle (3), un canal de liaison (24, 34) étant prévu dans l'articulation à rotule, lequel relie un espace intérieur (390) de la téterelle (3) à un espace intérieur (11) du récipient de collecte de lait (1) afin de guider le lait depuis la téterelle (3) jusque dans le récipient de collecte de lait (1, 2), **caractérisée en ce qu'**une position de la téterelle (3) est prévue, dans laquelle ce canal de liaison (24, 34) s'étend dans la direction du deuxième axe médian longitudinal (b), et **en ce que** soit
a) l'articulation à rotule présente une rotule d'articulation (31) qui est façonnée ou disposée sur la téterelle (3), l'articulation à rotule présentant un logement de rotule (21) qui est façonné ou disposé dans un couvercle (2) du récipient de collecte de lait (1, 2), soit
b) l'articulation à rotule présente une rotule d'articulation (23) qui est façonnée ou disposée sur un couvercle (2) du récipient de collecte de lait (1, 2).

2. Unité de tirage de lait selon la revendication 1, dans laquelle le récipient de collecte de lait présente une partie de récipient (1) et un couvercle (2) fermant cette partie de récipient (1).

3. Unité de tirage de lait selon l'une quelconque des revendications 1 ou 2, dans laquelle la première partie d'accouplement (31) est façonnée d'une seule pièce au niveau d'une partie de la téterelle (3) et/ou la deuxième partie d'accouplement (21, 23) est façonnée d'une seule pièce au niveau d'une partie du récipient de collecte de lait (1, 2).

4. Unité de tirage de lait selon l'une quelconque des revendications 1 ou 2, dans laquelle au moins l'une des deux parties d'accouplement (6) est une partie supplémentaire au récipient de collecte de lait (1, 2) et à la téterelle (3), la partie d'accouplement supplémentaire (6) présentant, dans la direction de la connexion entre le récipient de collecte de lait (1, 2) et la téterelle (3), une longueur qui est considérablement inférieure à la hauteur du récipient de collecte de lait (1, 2) et/ou de la téterelle (3).

5. Unité de tirage de lait selon l'une quelconque des revendications 1 à 4, dans laquelle le récipient de collecte de lait (1, 2) présente une section transversale en forme de haricot.

6. Téterelle (3) pour une unité de tirage de lait selon l'une quelconque des revendications 1 à 5, dans laquelle la téterelle (3) définit un premier axe médian longitudinal (a) et présente une première partie d'accouplement (31) qui permet un mouvement relatif entre la téterelle (3) et un récipient de collecte de lait (1, 2) dans un état dans lequel le récipient de collecte de lait (1, 2) est fixé à la téterelle (3), le récipient de collecte de lait (1, 2) définissant un deuxième axe médian longitudinal (b), la première partie d'accouplement (31) pouvant être raccordée à une deuxième partie d'accouplement (21, 23) du récipient de collecte de lait (1, 2), les deux parties d'accouplement permettant ensemble le mouvement relatif, l'angle entre le premier axe médian longitudinal (a) et le deuxième axe médian longitudinal (b), dans l'état fixé du récipient de collecte de lait (1, 2), pouvant être modifié, la première partie d'accouplement (31) formant avec la deuxième partie d'accouplement (21, 23) une articulation à rotule, un canal de liaison (24, 34) étant prévu dans l'articulation à rotule, lequel relie un espace intérieur (390) de la téterelle (3) à un espace intérieur (11) du récipient de collecte de lait (1), afin de conduire du lait depuis la téterelle (3) jusque dans le récipient de collecte de lait (1, 2), la première partie d'accouplement présentant une rotule d'articulation (31) ou un logement de rotule (21) de l'articulation à rotule et la deuxième partie d'accouplement (21, 23) présentant un logement de rotule correspondant (21) ou une rotule d'articulation (31) de l'articulation à rotule et une position de la téterelle (3) étant prévue, dans laquelle ce canal de liaison (24, 34) s'étend dans la direction du deuxième axe médian longitudinal (b), **caractérisée en ce qu'**une position de la téterelle (3) est prévue, dans laquelle le canal de liaison (24, 34) s'étend dans une direction qui forme un angle avec le premier axe médian longitudinal (a).

7. Récipient de collecte de lait (1, 2) destiné à l'utilisation dans une unité de tirage de lait selon l'une quelconque des revendications 1 à 5, le récipient de collecte de lait (1, 2) étant réalisé sous forme stable ou approximativement sous forme stable et présentant une deuxième partie d'accouplement (21, 23) qui permet un mouvement relatif entre le récipient de collecte de lait (1, 2) et une téterelle (3) dans un état dans lequel le récipient de collecte de lait (1, 2) est fixé à la téterelle (3), le récipient de collecte de lait (1, 2) définissant un deuxième axe médian longitudinal (b), la deuxième partie d'accouplement (21, 23) pouvant être connectée à une première partie d'accouplement (31) de la téterelle (3), les deux parties d'accouplement permettant ensemble le mouvement relatif, l'angle entre le premier axe médian longitudinal (a) et le deuxième axe médian longitudinal (b) dans l'état fixé du récipient de collecte de lait (1, 2) pouvant être modifié, la première partie d'accouplement (31) formant avec la deuxième partie d'accouplement (21, 23) une articulation à rotule, un canal de liaison (24, 34) étant prévu dans l'articulation à rotule, lequel relie un espace intérieur (390) de la téterelle (3) à un espace intérieur (11) du récipient de collecte de lait (1), afin de conduire du lait depuis la téterelle (3) jusque dans le récipient de collecte de lait (1, 2), la première partie d'accouplement présentant une rotule d'articulation (31) ou un logement de rotule (21) de l'articulation à rotule et la deuxième partie d'accouplement (21, 23) présentant un logement de rotule correspondant (21) ou une rotule d'articulation (31) de l'articulation à rotule et une position de la téterelle (3) étant prévue, dans laquelle le canal de liaison (24, 34) s'étend dans la direction du deuxième axe médian longitudinal (b).

8. Unité d'accouplement (31, 21 ; 23) pour l'utilisation dans une unité de tirage de lait selon l'une quelconque des revendications 1 à 5, l'unité d'accouplement (31, 21 ; 23) présentant une première et une deuxième partie d'accouplement pour la connexion d'une téterelle (3) avec un premier axe médian longitudinal (a) et d'un récipient de collecte de lait de forme stable ou approximativement de forme stable (1, 2) avec un deuxième axe médian longitudinal (b), la première et la deuxième partie d'accouplement permettant ensemble un mouvement relatif entre la téterelle (3) et le récipient de collecte de lait (1, 2) fixé à la téterelle (3), l'angle entre le premier axe médian longitudinal (a) et le deuxième axe médian longitudinal (b) dans l'état du récipient de collecte de lait (1, 2) fixé à la téterelle (3) pouvant être modifié, la première partie d'accouplement (31) formant avec la deuxième partie d'accouplement (21, 23) une articulation à rotule, un canal de liaison (24, 34) étant prévu dans l'articulation à rotule, lequel relie un espace intérieur (390) de la téterelle (3) à un espace intérieur (11) du récipient de collecte de lait (1), afin de guider le lait depuis la téterelle (3) jusque dans le récipient de collecte de lait (1, 2) et la première partie d'accouplement présentant une rotule d'articulation (31) ou un logement de rotule (21) de l'articulation à rotule et la deuxième partie d'accouplement (21, 23) présentant un logement de rotule correspondant (21) ou une rotule d'articulation (31) de l'articulation à rotule et une position de la téterelle (3) étant prévue, dans laquelle ce canal de liaison (24, 34) s'étend dans la direction du deuxième axe médian longitudinal (b).
